# EUROPEAN PATENT APPLICATION

(11) **EP 1 637 070 A2**
(43) Date of publication of application: **22.03.2006**
(21) Application number: 05255499.5
(22) Date of filing: 08.09.2005
(51) Int. Cl.: A61B 5/0205, A61B 8/00

(54) **Integrated anesthesia monitoring and ultrasound display**

(30) Priority: 16.09.2004 US 610473 P; 01.08.2005 US 194331
(71) Applicant: GENERAL ELECTRIC COMPANY, Schenectady, New York 12345 (US)
(72) Inventor: Sandy, Neal J., Middleton, Wisconsin 53562 (US); Raz, Israel, Highland Park, Illinois 60035 (US); Haggblom, Tom J., Vantaa 01520 (FI)
(74) Representative: Pedder, James Cuthbert

(57) **Abstract**

An apparatus and method for synchronizing the hemodynamic measurements (36,38,40) from a patient monitoring system (46) and the ultrasound images (32) from an ultrasound monitoring system (44). The synchronized images and measurements can be combined and displayed on a single display (48) that includes both the ultrasound images and the hemodynamic patient measurements. The combined display receives synchronized information (50) from both the ultrasound monitoring system and the hemodynamic patient monitoring system and displays the combined information on a single monitor. In addition to displaying the synchronized combined information, the system stores the synchronized information for later playback. The playback of the stored images and measurements is synchronized such that the clinician can be presented with the combined information in a usable manner.

## Description

The present invention relates to a cardiovascular care system that synchronizes the monitoring information from two cardiac monitoring devices and presents the information for viewing and analysis. More specifically, the present invention relates to a synchronization and storage system that combines information from an ultrasound monitoring system and a traditional hemodynamic patient monitoring unit, such as is used during anesthesia procedures.

In modern operating rooms, anesthesiologists and surgeons utilize cardiovascular ultrasound units to monitor heart function during surgery. In cases in which the anesthesiologist determines that a patient's heart condition is weak or at risk, the clinician will monitor the heart using ultrasound in addition to a traditional hemodynamic heart function measurements taken using an anesthesia monitoring system. Additionally, the ultrasound units are also often used perioperative (before, during and after surgery) for cardiac ultrasound diagnostic evaluation for cardiac surgeries, such as valve repair, heart bypass, transplants, etc. In such cases, the surgeon relies heavily on ultrasound images and measurements to make an accurate diagnosis and monitor the patient during the procedure.

Currently, traditional hemodynamic anesthesia monitoring equipment includes a display and various operating components mounted to a single movable cabinet that can be positioned in a desirable location in the operating room. The traditional hemodynamic anesthesia monitoring unit allows the clinician to accurately monitor the status of the patient during surgery, including the hemodynamic measurements of the patient's blood pressure, oxygen saturation, heart rate and electrical heart activity (ECG). Typically, the monitoring unit is positioned at a location in the operating room that can be easily viewed by the clinician during the surgical procedure.

As discussed above, in the modern operating room, the use of a cardiovascular ultrasound unit to selectively monitor the patient's heart function has become more common. Typically, an ultrasound unit is contained in a transportable cart and includes a display showing the current ultrasound images. As with the anesthesia monitoring unit, the ultrasound unit includes a display and operating components on a platform that can be positioned for viewing within the operating room. Although the position of the two monitoring units can be adjusted within the operating room, the available space in most operating rooms severely limits the positioning of both the ultrasound unit and the anesthesia monitoring unit. In many cases, the anesthesia monitoring unit and the ultrasound unit are placed opposite sides of the clinician, thereby requiring the clinician to turn his attention away from either the surgical procedure or the anesthesia monitoring unit in order to view the ultrasound display.

In many cases, it is desirable for the clinician to view both the ultrasound images and the heart waveforms simultaneously so that the clinician can monitor the current status of the patient. In currently available equipment, the patient monitoring display does not show the ultrasound images, while the ultrasound display does not include the hemodynamic measurements desired by the clinician. Thus, the clinician must constantly shift his or her attention between the two separate displays of the anesthesia patient monitoring equipment and the ultrasound unit.

The use of separate anesthesia patient monitoring and ultrasound monitoring systems presents several different challenges to a clinician. As described, since space in the operating room is at a premium, the size of a traditional ultrasound system occupies considerable real estate near the surgical table. Further, since a traditional ultrasound system can weigh up to 400 Ibs., moving the ultrasound system from one operating room suite to another during surgery can result in tripping over or damaging cables or other instruments. Moving systems between operating rooms is practiced often even during surgery, as several hours may pass until the system is needed at the end of a procedure, and is used meanwhile in a different case elsewhere.

One of the significant challenges of utilizing separate anesthesia patient monitoring systems and ultrasound systems is the time-based synchronization of the two displays when reviewing stored information. For example, the meaningful interpretation of ultrasound abnormalities, such as mitral regurgitation, can be accomplished only in the context of other time-synchronous hemodynamic parameters such as blood pressure. Presently, the stored images from the ultrasound unit are time-stamped and stored either in the ultrasound unit or at a remote location (e.g., server). The time data stored with the ultrasound images is useful in reviewing the stored images at a later time or from a remote location, such as by trained technicians or interpreting physicians. Likewise, the hemodynamic measurements from the anesthesia monitoring unit are also stored and include time-based information such that the stored data can be reviewed at a later time or from a remote location by trained technicians. However, the stored images from the ultrasound unit and the hemodynamic measurements from the anesthesia patient monitoring system are not integrated with each other, such that the time-based stored information is not correlated or synchronized to the other unit.

The present invention is a system and method that integrates the functionality of an ultrasound scanning system and a patient monitoring system, such as an anesthesia monitoring system. Preferably, the integration will occur at three different levels. First, the ultrasound system and the anesthesia monitoring system may be mechanically coupled such that both displays are on the same plane of view, with the ultrasound system receiving the hemodynamic monitoring information or vice versa. This mechanical coupling will allow the clinician to reduce the clutter within an operating room theater.

Second, the integrated system may include a single display that shows a user-selected combination of selected ultrasound images and selected hemodynamic monitoring parameters. The combined display may occur on the patient monitor display, the traditional ultrasound display or on a new display designed for the combined image.

Third, the integrated system allows the monitored data from both the ultrasound and anesthesia monitoring systems to be saved digitally and with time synchronization such that the hemodynamic measurements can accurately be correlated with the heart function as depicted by the synchronized ultrasound image. The synchronization of the hemodynamic measurements and the ultrasound images allows a clinician to review the monitored information at a later time or simultaneously to the data/image acquisition from a remote location and obtain the required information from the combined data.

Preferably, the ultrasound monitoring and the anesthesia monitoring systems include a communication link that allows the ultrasound monitoring system and the anesthesia monitoring system to be synchronized with each other. The synchronization of the ultrasound monitoring system and the anesthesia monitoring system allows the images from the ultrasound system and the hemodynamic measurements from the anesthesia monitoring system to be time synchronized. The ultrasound images and hemodynamic measurements can then be stored at a common location or in separate databases and later retrieved for viewing.

As an example, the ultrasound images and the hemodynamic measurements from the anesthesia monitoring system can be stored in a central database located remotely from both the ultrasound monitoring system and the anesthesia monitoring system. Alternatively, the central database can be incorporated into the anesthesia monitoring system or the ultrasound monitoring system. In either case, the hemodynamic measurements from the anesthesia monitoring system and the images from the ultrasound system each include synchronization data from a common source such that each system is synchronized with the other system.

After both the hemodynamic measurements and the ultrasound images are stored, a clinician can review the ultrasound images and the hemodynamic measurements at a later time or from a remote location. Preferably, the ultrasound images and the hemodynamic measurements will be displayed on an integrated display device. However, the use of multiple displays to show the time synchronized information from the ultrasound monitoring system and the anesthesia monitoring system allows the clinical to obtain a complete understanding of the patient's condition at a time either before or after the ultrasound images are recorded.

It is contemplated that the ultrasound monitoring system and the anesthesia monitoring system can store and record continuous information from the patient at either local or remote storage locations. Alternatively, the storage of the ultrasound images and the hemodynamic measurements can be selectively triggered by the clinician when a triggering event occurs during the monitoring window. As an example, the clinician can depress a record button or similar component on the ultrasound monitoring system when the triggering event occurs, which then triggers the storage of hemodynamic patient information from the anesthesia monitoring system at a period of time immediately before and immediately following the triggering event on the ultrasound monitoring system.

The anesthesia monitoring system and the ultrasound monitoring system can also receive synchronization information from a common, remote location such that the anesthesia monitoring system and the ultrasound monitoring system do not need to be directly connected. In this type of combined system, a central controller and/or database provides time synchronization signals to the anesthesia monitoring system and the ultrasound monitoring system, where the central database receives synchronization data and the desired information from both of the two monitoring systems. The stored information from the central database can then be viewed either locally or through a remote terminal connected to the central database by a WAN, LAN, or Wireless technologies.

Although the present invention is particularly useful in the operating room with an anesthesia monitoring system, the combined display would be equally desirable with other types of patient monitoring systems, such as in acute, perinatal and critical care environments, as well as physicians' office and reviewing rooms.

The invention will now be described in greater detail, by way of example, with reference to the drawings, in which:-
Figure 1 is a screen display of the traditional hemodynamic patient monitor;
Figure 2 is a conventional display of an ultrasound monitoring system;
Figure 3 is a combined display of ultrasound imaging and hemodynamic monitoring information and waveforms of the present invention;
Figure 4 is a contemplated embodiment of the combined anesthesia monitoring system and ultrasound monitoring system;
Figure 5 is a schematic illustration of the interconnections between the anesthesia monitoring system and ultrasound monitoring system that allows the information from both systems to be synchronized and stored;
Figure 6 is a schematic illustration of a second embodiment of the invention interconnections between the anesthesia monitoring system and the ultrasound monitoring system that allows the information from both systems to be synchronized and stored;
Figure 7 is a schematic illustration of another embodiment of the interconnection between the anesthesia monitoring system and the ultrasound monitoring system that allows the information from both systems to be synchronized and stored;
Figure 8 is a schematic illustration of another embodiment of the interconnection between the anesthesia monitoring system and the ultrasound monitoring system that allows the information from both systems to be synchronized and stored; and
Figure 9 is a schematic illustration of another embodiment of the interconnection between the anesthesia monitoring system and the ultrasound monitoring system that allows the information from both systems to be synchronized and stored.

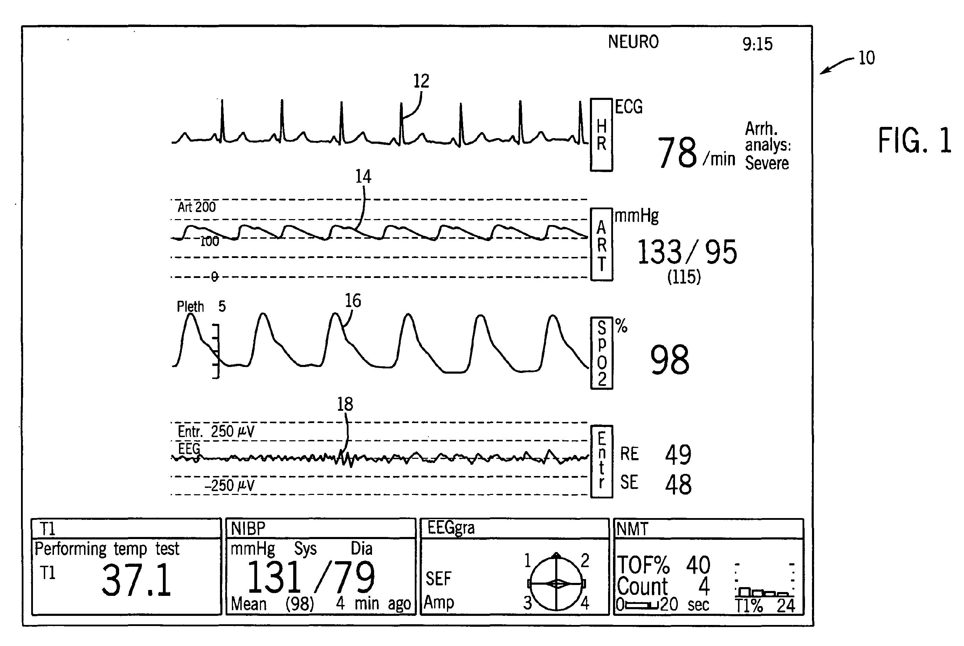

Referring first to Figure 1, there is shown a conventional display 10 that is typically found in a hemodynamic patient monitoring system. The hemodynamic patient monitoring system has a variety of different uses and is widely used in the operating room during procedures requiring the use of anesthesia. As illustrated in Figure 1, the standard anesthesia display 10 graphically depicts a series of hemodynamic measurements including at least one ECG trace 12, an invasive blood pressure trace 14, an oxygen saturation trace 16 and an entropy trace 18. Each of the traces 12-16 is made and presented on the display 10 in real time and presents the user with the required information to monitor the patient during the anesthesia procedure. The individual traces 12-18 are conventional hemodynamic measurements and are widely utilized in the operating room environment.

Although the following description will make particular reference to a hemodynamic anesthesia monitoring system, it should be understood that the anesthesia monitoring system could be any type of patient monitoring system, such as those used in acute, perinatal, neo-natal and/or critical care environments. In each situation, the patient monitoring system, such as the anesthesia monitoring system, monitors vital sign data from the patient and presents the monitored information for analysis by a physician or a clinician. Thus, throughout the remaining description, the anesthesia monitoring system could be replaced with other types of patient monitoring systems while operating within the scope of the present invention.

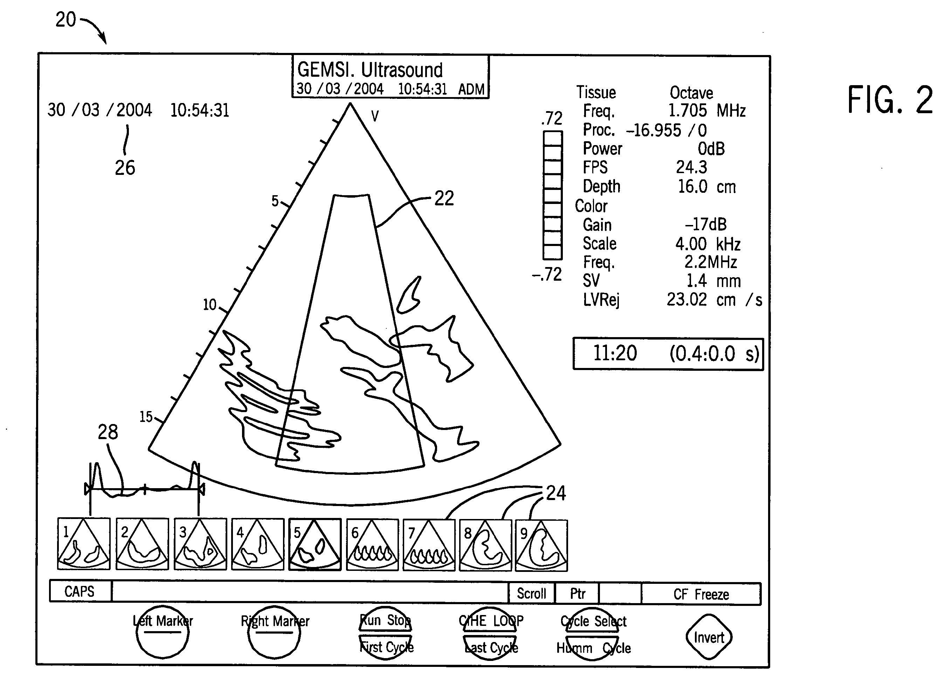

During many cardiac surgery procedures, it is desirable to utilize a cardiac ultrasound monitoring system to monitor the performance of the patient's heart during the procedure. A typical ultrasound monitoring display is shown in Figure 2 as reference numeral 20. In the ultrasound display 20, the display includes the ultrasound image 22 as well as various different image views 24 that can be selected by the operating room personnel. The ultrasound image includes a time display 26 that allows the clinician to determine the actual time the image being displayed was taken, particularly when an archived image is replayed on the display 20. As illustrated in the ultrasound display 20, the ultrasound system includes a rudimentary heart rate graph 28 that allows the clinician to analyze the ultrasound image 22 relative to the time position of the image within the patient's heart beat cycle.

As discussed previously, during many cardiac procedures, both the anesthesiologist and the surgeon desires to view the hemodynamic measurements included on the anesthesia monitoring system display 10 and the ultrasound images on the ultrasound display 20 during the surgical procedure. However, since the ultrasound unit and the anesthesia monitoring system are two separate devices that include their own separate, independent displays, such viewing requires the user to shift attention between the two displays. As described, in the operating room theater, the anesthesiologist or surgeon must constantly shift their attention between the two separate displays in order to acquire the information needed to either perform a procedure or analyze the current situation.

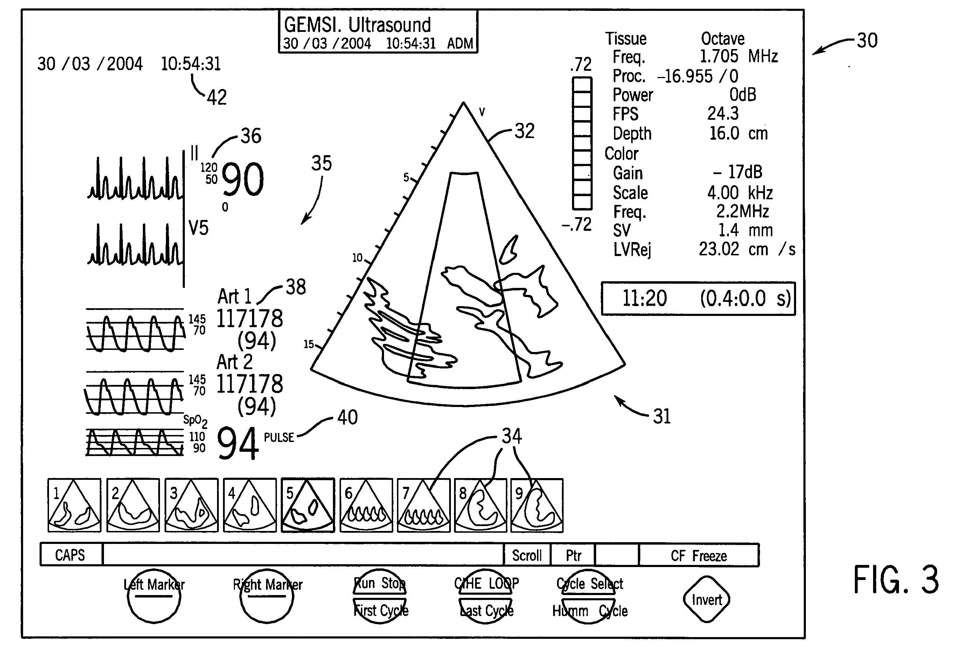

Referring now to Figure 3, there is shown a combined ultrasound and hemodynamic anesthesia monitoring display 30. As illustrated in Figure 3, the combined display 30 includes an ultrasound image viewing section 31 that includes the ultrasound image 32 and the plurality of thumb nail images 34. The thumb nail images 34 allow the operator to select between different views available from the ultrasound system.

In addition to the ultrasound image viewing section 31, the combined display 30 includes a hemodynamic display section 35 that includes selected hemodynamic measurements, including a pair of ECG traces 36, a pair of invasive blood pressure traces 38 and an oxygen saturation trace 40. The hemodynamic data for the ECG trace 36, blood pressure trace 38 and oxygen saturation trace 40 is received from the anesthesia monitoring system, while the ultrasound image 32 and the thumb nail images 34 are received from the ultrasound monitoring system. Although three traces 36-40 are shown on the combined display, other information could also be included on the combined display depending on the user requirements.

As illustrated in Figure 3, the combined display includes a time display 42 that is used to synchronize the images received from the ultrasound monitoring system and the hemodynamic measurements from the anesthesia monitoring system. The use of the single time display 42 allows the images and the monitored data to be synchronized and stored. The synchronization of the stored information allows the monitored data and images to be retrieved from a storage location at a later date and be presented in a synchronized manner, such as is shown in Figure 3. The synchronization of the ultrasound images and the hemodynamic measurements allows a clinician to review the combined monitoring results in a useful and time efficient manner.

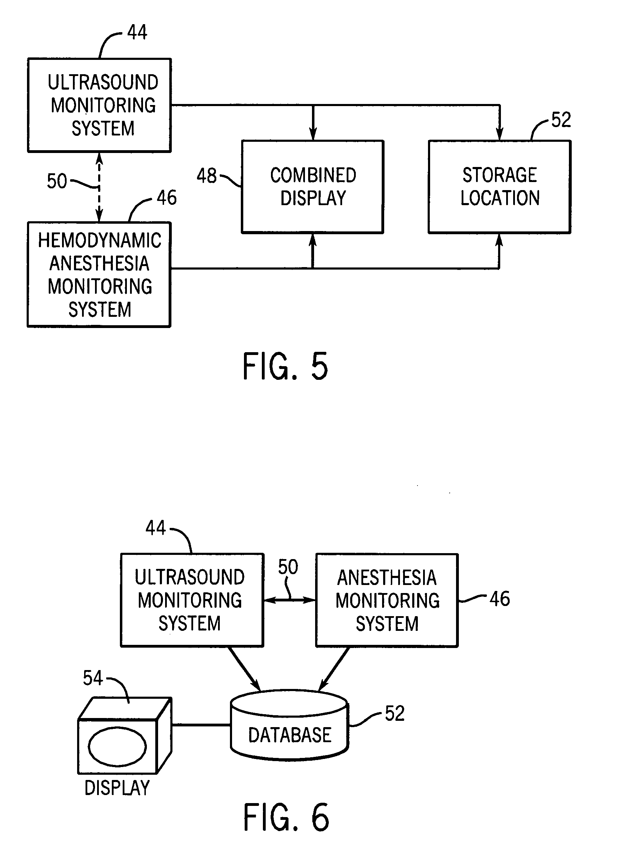

Referring now to Figure 5, there is shown a schematic illustration of the communication between the ultrasound monitoring system 44 and the anesthesia patient monitoring system 46. As illustrated, both the ultrasound monitoring system 44 and the anesthesia monitoring system 46 communicate their monitored information to a combined display 48. It is contemplated that the combined display 48 could either be the display included in the ultrasound monitoring system 44, the display in the hemodynamic anesthesia monitoring system 46 or a separate, third display that receives information from both the ultrasound monitoring system 44 and the hemodynamic anesthesia monitoring system 46. Alternatively, the combined display 48 could replace the displays used in both the ultrasound monitoring system 44 and the hemodynamic anesthesia monitoring system 46. In either case, the information from the ultrasound monitoring system 44 and the anesthesia monitoring system 46 is combined and displayed in a single location, such as in the manner shown in Figure 3.

As illustrated in Figure 5, a synchronization signal 50 is used between the ultrasound monitoring system 44 and the hemodynamic anesthesia monitoring system 46 to synchronize the internal clocks of each system. The synchronization signal 50 allows the information from each of the systems 44, 46 to be stored at either a common location 52 or separate locations where both the ultrasound images and the hemodynamic measurements include the same synchronization data. The synchronization of the information from the ultrasound system 44 and the hemodynamic anesthesia monitoring system 46 allows the information to be retrieved and displayed at a later time such that the information is time-synchronized and can be reviewed by a clinician. The ability of a clinician to view the ultrasound and hemodynamic information in synchronization allows the clinician to be presented with a complete picture of the procedure, which was currently unavailable.

In addition to the embodiment illustrated in Figure 5, various other configurations of the system of the present invention are currently contemplated. In each case, the ultrasound images created by the ultrasound monitoring system 44 and the hemodynamic measurements from the anesthesia monitoring system 46 are synchronized with each other and stored for viewing at a later date. Since the ultrasound images and the hemodynamic measurements are synchronized and stored with synchronization data, the ultrasound images and hemodynamic measurements can be retrieved and viewed at a later date in synchronization with each other. The synchronization of the ultrasound images and the patient waveforms allows a clinician to study the ultrasound images and the hemodynamic measurements at the time of the ultrasound images, as well as before and immediately following the ultrasound images. The use of the hemodynamic measurements from the anesthesia monitoring system in synchronization with the ultrasound images presents the clinician a better overall summary of the patient condition.

Referring now to Figure 6, in the embodiment of the invention illustrated, the ultrasound monitoring system 44 and the anesthesia monitoring system 46 each communicate with a central database 52. Since the ultrasound monitoring system 44 and the anesthesia monitoring system 46 receive the common synchronization signal 50, the ultrasound images and hemodynamic measurements stored at the central database 52 are synchronized with each other. The central database 52 can be any type of storage media, including part of a hospital information system (HIS). A remote display terminal 54 can be in communication with the central database 52 to retrieve and display the images from the ultrasound monitoring system 44 and the hemodynamic measurements from the anesthesia monitoring system 46. The measurements and ultrasound images can be simultaneously displayed in synchronization for analysis by the clinician.

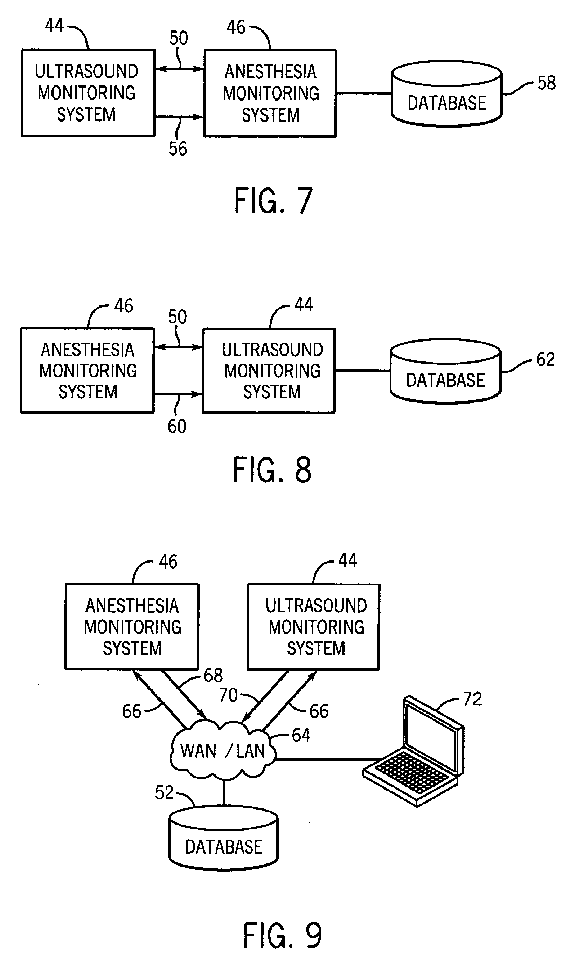

Referring now to Figure 7, the ultrasound monitoring system 44 and the anesthesia monitoring system 46 again receive the common synchronization signal 50. However, in the embodiment illustrated, the ultrasound monitoring system 44 communicates ultrasound images to the anesthesia monitoring system 46, as illustrated by arrow 56. The ultrasound images are received by the anesthesia monitoring system 46 and stored within a database 58 that is included as part of the anesthesia monitoring system 46. The database 58 can be accessed by the anesthesia monitoring system 46 or could be accessed by a remote terminal or other type of data acquisition and display device. Since the anesthesia monitoring system 46 typically includes a database 58, the database 58 can be used for storing not only the hemodynamic measurements, but also the ultrasound images.

Referring now to Figure 8, there is shown yet another embodiment of the invention in which the anesthesia monitoring system 46 and the ultrasound monitoring system 44 are synchronized through the synchronization signal 50. In this embodiment, the hemodynamic measurements from the anesthesia monitoring system 46 are transferred to the ultrasound monitoring system 44, as illustrated by arrow 60. The ultrasound images and hemodynamic measurements are stored in a database 62 included as part of the ultrasound system 44. Like the embodiment illustrated in Figure 7, the database 62 can be accessed either through the ultrasound monitoring system 44 or through an external data acquisition and display device. Typically, the ultrasound monitoring system 44 includes the internal database 62 such that no additional components are required to store the received information from the anesthesia monitoring system 46.

As can be understood in the embodiments of the invention illustrated in Figures 6-8, the ultrasound monitoring system 44 and the anesthesia monitoring system 46 include a communication interface that allows the two systems 44, 46 to communicate with each other. Typically, when either of the systems 44, 46 is used with a patient, patient demographic information needs to be either entered into the monitoring system or uploaded from the hospital information system (HIS). Since the ultrasound monitoring system 44 and the anesthesia monitoring system 46 are in communication with each other, the patient demographic data can be shared between the ultrasound monitoring system 44 and the anesthesia monitoring system 46. The shared patient demographic information eliminates the requirement that the clinician/physician enter duplicate information into each of the two monitoring systems 44, 46. Additionally, the communication between the two monitoring systems 44, 46 requires that only one of the ultrasound monitoring system 44 and the anesthesia monitoring system 46 be connected to the hospital information system, thereby possibly eliminating a data connection.

Figure 9 illustrates yet another contemplated embodiment of the invention. In this embodiment, both the anesthesia monitoring system 46 and the ultrasound monitoring system 44 communicate to the central database 52 through some type of computer network 64. In the embodiment of the invention illustrated in Figure 9, the network 64 is either a wide area network (WAN) or a local area network (LAN). In the embodiment of the invention illustrated in Figure 9, both the anesthesia monitoring system 46 and the ultrasound monitoring system 44 receive a synchronization signal 66 from the network 64. The synchronization signals 66 received from the network 64 are in time synchronization such that the ultrasound images and hemodynamic measurements are each synchronized, even though no direct communication is present between the anesthesia monitoring system 46 and the ultrasound monitoring system 44. Alternatively, the ultrasound monitoring system 44 and the anesthesia monitoring system 46 can include direct communication between each other to synchronize the ultrasound images and hemodynamic waveforms obtained by the monitoring systems.

The anesthesia monitoring system 46 relays information, as shown by arrow 68, through the network 64 to the central database 52. Likewise, the ultrasound monitoring system 44 is relaying ultrasound images, as shown by arrow 70, to the same central database 52. The ultrasound images and hemodynamic measurements are received and stored at the central database 52 and are synchronized with each other and can be accessed remotely through a remote terminal 72. The remote terminal 72 communicates with the central database 52 through the network 64 using either a hard wired or wireless interconnection. The remote terminal 72 can be used by a clinician or physician to monitor the ultrasound images and hemodynamic measurements in near real time, either by communicating directly with the anesthesia monitoring system 46 and the ultrasound monitoring system 44 through the network 64 or by communicating with the database 52 through the network 64. In this manner, a physician located remotely from the patient can monitor the synchronized ultrasound images and hemodynamic measurements. Further, it is contemplated that one or both of the anesthesia monitoring system 46 and the ultrasound monitoring system 44 could communicate to the network 64 using wireless communication techniques.

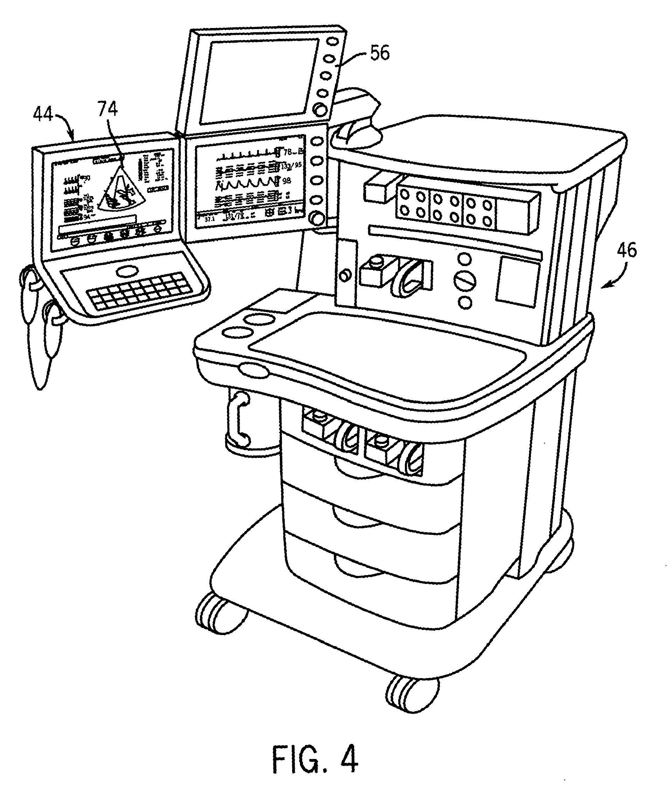

Referring now to Figure 4, there is shown a combined hemodynamic anesthesia monitoring system 46 and an ultrasound monitoring system 44. In the embodiment of the invention shown in Figure 4, the ultrasound display 74 is configured to receive information from the hemodynamic anesthesia monitoring system 46 to create the combined display as shown in Figure 3. As can be understood in Figure 4, the combined display on the ultrasound display 74 allows the clinician to view the required information without having to look at multiple monitors. In the embodiment of the invention shown in Figure 4, the anesthesia monitor includes its own display 76 to generate the images shown in Figure 1. However, the information from the display 76 is also shown on display 74 such that the clinician does not need to view both displays to understand the complete information being acquired from the patient at a given time. Further, the combined system of Figure 4 allows the ultrasound system 44 and the hemodynamic anesthesia monitor 46 to be located in the same position and move between operating room theaters.

In the combined anesthesia monitoring system and ultrasound monitoring system shown in Figure 4, the ultrasound monitoring system 44 will typically include some type of actuation button or switch that, when depressed, saves a series of ultrasound images within an internal database. In accordance with the present invention, the anesthesia monitoring system 46 and the ultrasound monitoring system 44 include a time synchronization signal that allows the ultrasound images stored by the ultrasound monitoring system 44 to be synchronized with the hemodynamic measurements from the anesthesia monitoring system 46.

## Claims

1. A method of displaying ultrasound images from an ultrasound monitoring system and hemodynamic measurements from a patient monitoring system, the method comprising the steps of:
providing a synchronization signal (50) to both the ultrasound monitoring system (44) and the patient monitoring system (46);
receiving the ultrasound images (32) and time-based synchronization data from the ultrasound monitoring system;
receiving the hemodynamic measurements (36,38,40) and time-based synchronization data from the patient monitoring system, wherein the ultrasound images and the hemodynamic measurements include the same time-based synchronization data; and
collectively displaying the time-synchronized ultrasound images and the hemodynamic measurements.

2. The method of claim 1 wherein the ultrasound images (32) and the hemodynamic measurements (36,38,40) are received at an integrated display device (48), wherein the ultrasound images and the hemodynamic measurements are simultaneously displayed on the integrated display device.

3. The method of claim 1 further comprising the step of storing the ultrasound images (32) and the hemodynamic measurements (36,38,40) in a common database (52).

4. The method of claim 3 further comprising the step of displaying the stored ultrasound images (32) and hemodynamic measurements (36,38,40) on an integrated display device (48), wherein the ultrasound images and the hemodynamic measurements are simultaneously displayed on the integrated display device.

5. The method of claim 4 wherein the common database (52) and the integrated display device (48) are located separately from both the ultrasound monitoring (44) system and the patient monitoring system (46).

6. The method of claim 1 wherein the synchronization signal (50) is provided to both the ultrasound monitoring system (44) and the patient monitoring system (46) from a separate device.

7. An integrated display (48) for use in patient monitoring, the integrated display comprising:
an ultrasound image viewing section (31) for displaying an ultrasound image (32) taken of the patient; and
a hemodynamic display section (35) for displaying hemodynamic measurements (36,38,40) taken from the patient,
wherein the ultrasound image viewing section and the hemodynamic display section are shown on the integrated display simultaneously.

8. The integrated display of claim 7 wherein the integrated display (48) is configured to receive the ultrasound images (32) from an ultrasound monitoring system (44) and is configured to receive the hemodynamic measurements (36,38,40) from a patient monitoring system (46).

9. The integrated display of claim 7 wherein the ultrasound image viewing section (31) and the hemodynamic display section (35) display time-synchronized ultrasound images (32) and hemodynamic measurements (36,38,40).

10. The integrated display of claim 8 wherein the integrated display (48) is separate from the ultrasound monitoring system (44) and separate from the patient monitoring system (46).
